# EUROPEAN PATENT APPLICATION

(11) **EP 4 537 823 A1**
(43) Date of publication of application: **16.04.2025**
(21) Application number: 23819874.1
(22) Date of filing: 07.06.2023
(51) Int. Cl.: A61K 31/405, A61K 31/192, A61K 31/216, A61K 31/454, A61P 31/12

(54) **ANTIVIRAL AGENT**

(30) Priority: 10.06.2022 JP 2022094545
(71) Applicant: Tohoku University, Sendai-shi, Miyagi 980-8577 (JP)
(72) Inventor: ABE, Takaaki, Sendai-shi, Miyagi 980-8577 (JP); YASUDA, Jiro, Nagasaki-shi, Nagasaki 852-8521 (JP); SAKURAI, Yasuteru, Nagasaki-shi, Nagasaki 852-8521 (JP)
(74) Representative: Script Intellectual Property LLP
(86) International application number: PCT/JP2023/021188
(87) International publication number: WO 2023/238894

(57) **Abstract**

It is to provide an antiviral agent comprising, as an active ingredient, a low-molecular compound that can be produced relatively conveniently and inexpensively. The antiviral agent comprises one or more compounds selected from the group consisting of compounds of formula (I₀), formula (II) and formula (III) described below, and pharmaceutically acceptable salts thereof.

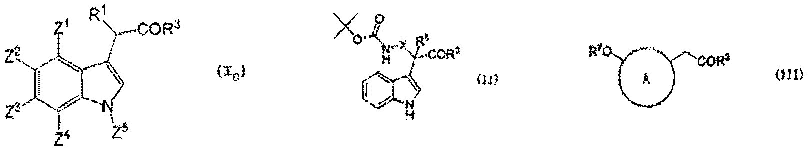

## Description

### Technical Field

The present invention relates to an antiviral agent.

### Background Art

Viruses are infectious parasites with a size of approximately 20 to 300 nm, and are composed mainly of protein shells (capsids) and genomes (RNAs or DNAs) inside the shells. After the viruses adsorb to host cells and invade the cells, the viral genomes are released (uncoated) and viral proteins and viral genomes are synthesized. The viruses are reconstructed based on the synthesized viral proteins and viral genomes, are released from the infected host cells and thereby further infects other host cells.

It has been reported that proanthocyanidin polyphenols derived from unripe apples or hop bracts have an antiviral activity (Patent Document 1). In addition, it has been reported, in Patent Document 2, that proanthocyanidin polymers obtained from Croton species exerts an antiviral effect (Patent Document 2).

On the other hand, the present inventors have reported that the present compound described below has an effect of enhancing erythropoietin expression and an effect of treating mitochondrial disease (Patent Document 3), an effect of inhibiting organ fibrosis (Patent Document 4), and an effect of preventing or ameliorating hearing loss (Patent Document 5). However, it has not heretofore been known that the present compound has an antiviral effect.

### Prior Art Documents

### Patent Documents

Patent Document 1: International Publication No. WO 2006/115123
Patent Document 2: Japanese Patent No. 3448052
Patent Document 3: International Publication No. WO 2014/080640
Patent Document 4: Japanese unexamined Patent Application Publication No. 2015-189670
Patent Document 5: Japanese unexamined Patent Application Publication No. 2019-116453

### Summary of the Invention

### Object to be Solved by the Invention

The object of the present invention is to provide an antiviral agent comprising, as an active ingredient, a low-molecular compound that can be produced relatively conveniently and inexpensively.

### Means to Solve the Object

The inventors have continued their diligent research to achieve the above object. In the course of the research, as a result of analyzing the antiviral effect using virus-infected cells, they have found that the present compound described below has an antiviral effect. The present invention has been completed based on these findings.

That is, the present invention is as follows:
[1] The antiviral agent comprising one or more compounds selected from the group consisting of a compound of formula (I₀), a compound of formula (II) and a compound of formula (III), and pharmaceutically acceptable salts thereof (hereinafter, sometimes referred to as "the present compound").

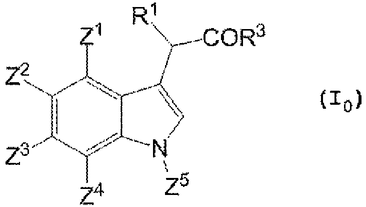

In formula (I₀),
R¹ represents
   a hydrogen atom;
   a benzoylmethyl group, the benzene ring of which is unsubstituted, or a benzoylmethyl group, the benzene ring of which is substituted with at least one group selected from the group consisting of an alkyl group having 1 to 7 carbon atoms, an alkoxy group having 1 to 7 carbon atoms, a fluorine atom and a chlorine atom;
   a linear or branched alkyl group, having 4 to 6 carbon atoms, unsubstituted or substituted with a fluorine atom;
   an alkyl group, having 1 to 6 carbon atoms, substituted with a group of formula (A), and optionally having an oxygen atom; or
   a methyl group or an ethyl group, substituted with a phenyl group, a cyclopentyl group or a 1-acetyl-4-piperidinyl group, the phenyl group being optionally further substituted with one or more phenyl groups;
Z¹, Z², Z³ and Z⁴ are the same or different and each represent a hydrogen atom, a halogen atom, a C1-C6 alkyl group, a C2-C6 alkenyl group, a C2-C6 alkynyl group or a group of OR⁸ wherein R⁸ represents a C1-C7 alkyl group, a C2-C6 alkenyl group or a C2-C6 alkynyl group;
Z⁵ represents a hydrogen atom or a C1-C10 alkyl group; and R³ represents any one group selected from the group consisting of OH, OR⁴, NHR⁴ and NR⁴R⁵, wherein R⁴ and R⁵ are the same or different and each represent a substituted or unsubstituted alkyl group having 1 to 4 carbon atoms.

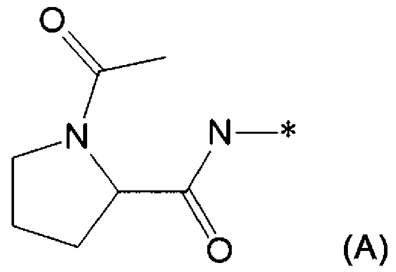

In formula (A), * indicates the bonding position.

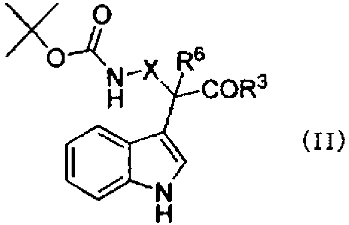

In formula (II),
R⁶ represents a hydrogen atom or a methyl group;
X represents an alkylene group, having 4 to 6 carbon atoms, optionally having an oxygen atom; and
R³ represents any one group selected from the group consisting of OH, OR⁴, NHR⁴ and NR⁴R⁵, wherein R⁴ and R⁵ are the same or different and each represent a substituted or unsubstituted alkyl group having 1 to 4 carbon atoms.

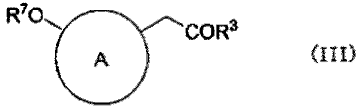

In formula (III),
A represents indole or naphthalene, and when A represents indole, it is substituted with a R³COCH₂ group and R⁷O at the 3-position and 5-position, respectively, and when A represents naphthalene, it is substituted with a R³COCH₂ group and R⁷O at the 1-position and 7-position, respectively; R⁷ represents an alkyl group having 1 to 10 carbon atoms or a benzyl group, the benzene ring of which is optionally substituted with one or more of an alkyl group having 1 to 3 carbon atoms or an alkoxy group having 1 to 3 carbon atoms; and
R³ represents any one group selected from the group consisting of OH, OR⁴, NHR⁴ and NR⁴R⁵, wherein R⁴ and R⁵ are the same or different and each represent a substituted or unsubstituted alkyl group having 1 to 4 carbon atoms.

[2] The antiviral agent according to [1] above, wherein the compound is a compound of formula (I₀) or a pharmaceutical acceptable salt thereof.

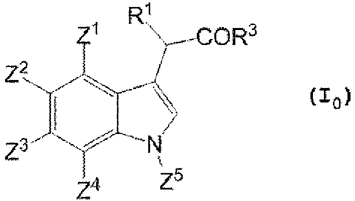

In formula (I₀),
R¹ represents
   a hydrogen atom;
   a benzoylmethyl group, the benzene ring of which is unsubstituted, or a benzoylmethyl group, the benzene ring of which is substituted with at least one group selected from the group consisting of an alkyl group having 1 to 7 carbon atoms, an alkoxy group having 1 to 7 carbon atoms, a fluorine atom and a chlorine atom;
   a linear or branched alkyl group, having 4 to 6 carbon atoms, unsubstituted or substituted with a fluorine atom;
   an alkyl group, having 1 to 6 carbon atoms, substituted with a group of formula (A), and optionally having an oxygen atom; or
   a methyl group or an ethyl group, substituted with a phenyl group, a cyclopentyl group or a 1-acetyl-4-piperidinyl group, the phenyl group being optionally further substituted with one or more phenyl groups;
Z¹, Z², Z³ and Z⁴ are the same or different and each represent a hydrogen atom, a halogen atom, a C1-C6 alkyl group, a C2-C6 alkenyl group, a C2-C6 alkynyl group or a group of OR⁸ wherein R⁸ represents a C1-C7 alkyl group, a C2-C6 alkenyl group or a C2-C6 alkynyl group;
Z⁵ represents a hydrogen atom or a C1-C10 alkyl group; and R³ represents any one group selected from the group consisting of OH, OR⁴, NHR⁴ and NR⁴R⁵, wherein R⁴ and R⁵ are the same or different and each represent a substituted or unsubstituted alkyl group having 1 to 4 carbon atoms.

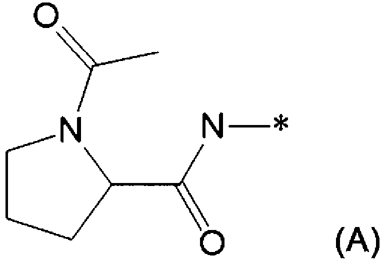

In formula (A), * indicates the bonding position. [3] The antiviral agent according to [1] above, wherein the compound is a compound of formula (I-2) (compound #5 described below in Examples), a compound of formula (I-3) (compound #1 described below in Examples), a compound of formula (I-4) (compound #7 described below in Examples), a compound of formula (I-5) (compound #9 described below in Examples), a compound of formula (I-6) (compound #18 described below in Examples), a compound of formula (I-7) (compound #19 described below in Examples), a compound of formula (I-8) (compound #21 described below in Examples), a compound of formula (I-9) (compound #22 described below in Examples), a compound of formula (I-10) (compound #30 described below in Examples), a compound of formula (II-2) (compound #13 described below in Examples) or a compound of formula (III-2) (compound #35 described below in Examples), or a pharmaceutically acceptable salt thereof.

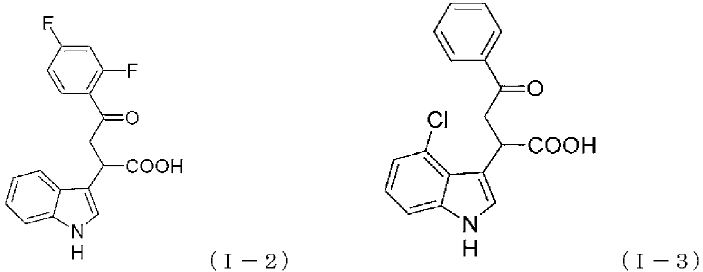

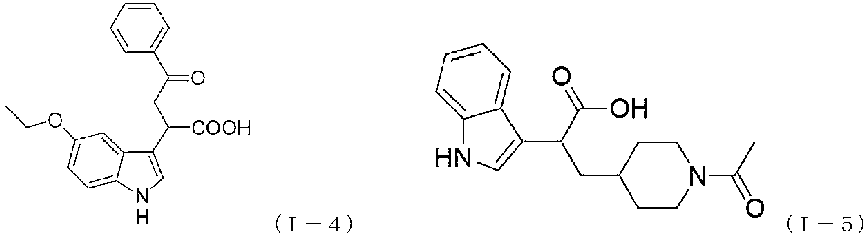

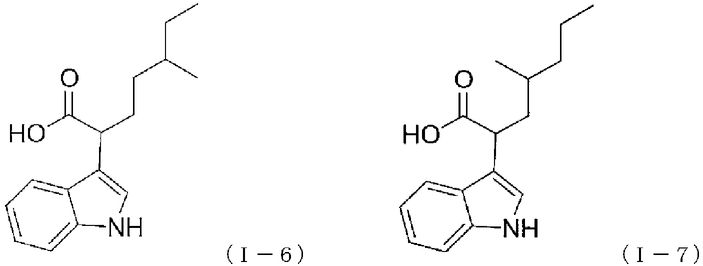

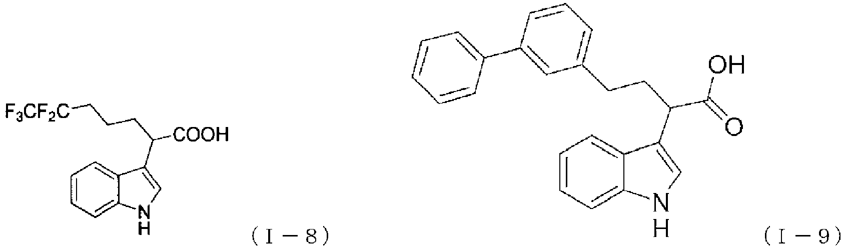

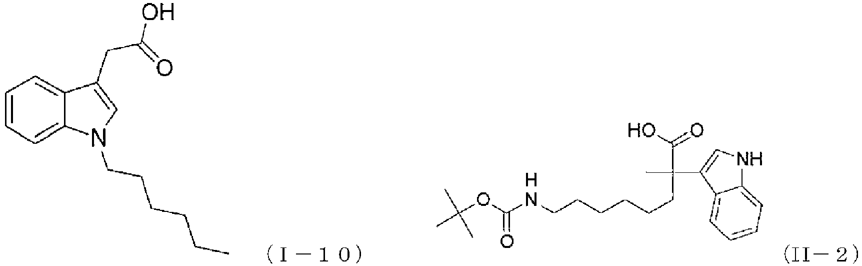

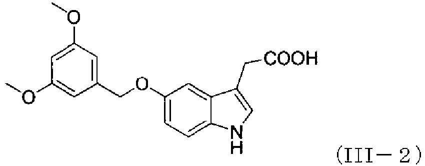

[4] The antiviral agent according to any one of [1] to [3] above, wherein the virus is coronavirus, influenza virus or Ebola virus.

Other aspects of the present invention include:
a method for preventing and/or treating a viral infection, comprising a step of administering the present compound to a subject (patient) in need of preventing and/or treating the viral infection;
the present compound for use as an antiviral agent;
the subject compound for use in antiviral purpose
the present compound for use in preventing and/or treating the viral infection;
use of the present compound for producing an antiviral agent; and
use of the present compound for producing an agent for preventing and/or treating a viral infection.

### Effect of the Invention

The present compound exerts an excellent antiviral effect. Therefore, the present invention is useful for the prevention or treatment of viral infections in the livestock industry and human medicine.

### Brief Description of Drawings

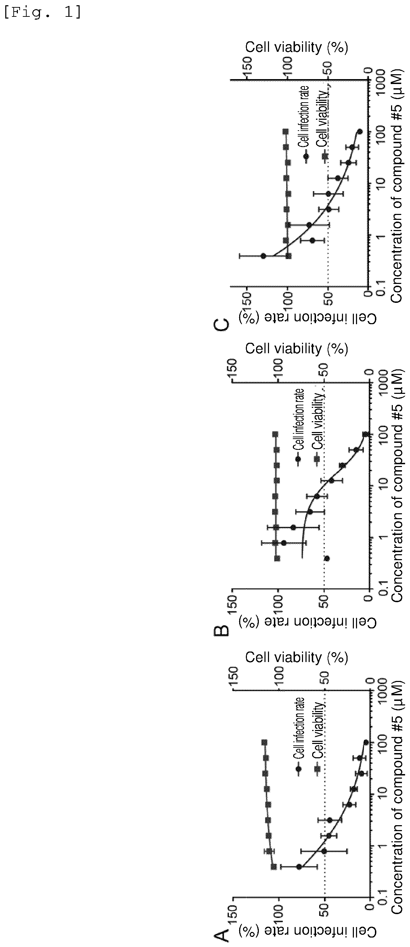

[Figure 1] Figure 1 is a graph showing the results of: infecting a human bronchial epithelial adenocarcinoma cell line (Calu-3 cell line) with 3 types of SARS-CoV-2 (a prototype having D614G mutation [Figure 1A], Omicron mutant BA.1 strain [Figure 1B] or Omicron mutant BA.2 strain [Figure 1C]) in the presence of compound #5; and analyzing the cell infection rate (%) and cell viability (%) after culturing for 3 days. Here, "cell infection rate (%)" refers to the percentage (%) of the number of infected cells under each condition to the number of infected cells when infected in the absence of compound #5, and "cell viability (%)" refers to the percentage (%) of the total number of viable cells under each condition to the total number of viable cells when infected in the absence of compound #5.

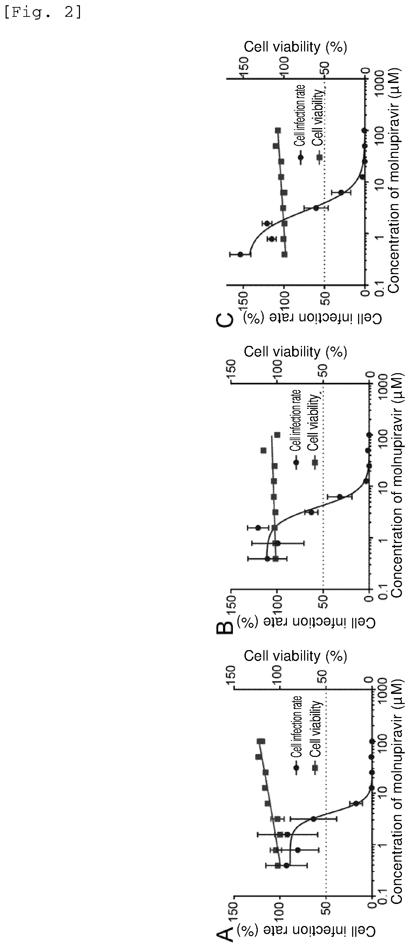

[Figure 2] Figure 2 is a graph showing the results of: infecting a Calu-3 cell line with 3 types of SARS-CoV-2 (a prototype having D614G mutation [Figure 2A], Omicron mutant BA.1 strain [Figure 2B] or Omicron mutant BA.2 strain [Figure 2C]) in the presence of molnupiravir; and analyzing the cell infection rate (%) and cell viability (%) after culturing for 3 days. Here, "cell infection rate (%)" refers to the percentage (%) of the number of infected cells under each condition to the number of infected cells when infected in the absence of molnupiravir, and "cell viability (%)" refers to the percentage (%) of the total number of viable cells under each condition to the total number of viable cells when infected in the absence of molnupiravir.

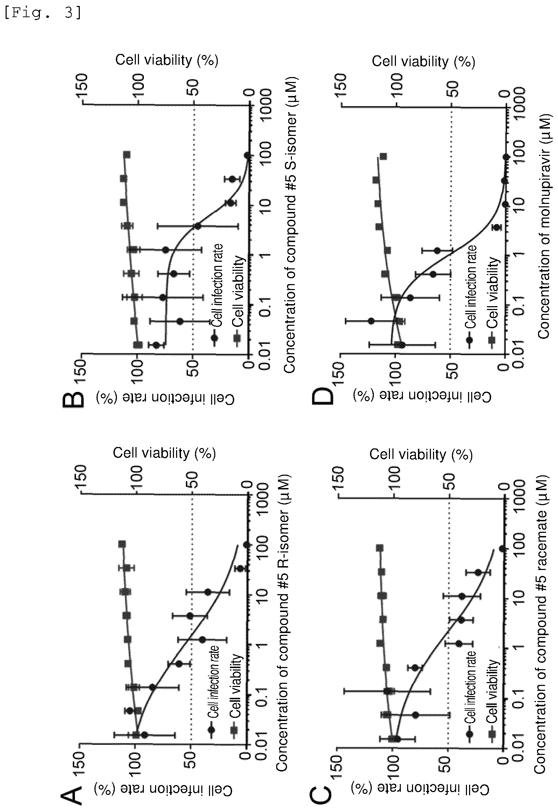

[Figure 3] Figure 3 is a graph showing the results of; infecting a Calu-3 cell line with SARS-CoV-2 (a prototype having D614G mutation) in the presence of 3 types of compound #5 (an R-isomer [Figure 3A], an S-isomer [Figure 3B] and a racemate [Figure 3C]) or molnupiravir (Figure 3D); and analyzing the cell infection rate (%) and cell viability (%) after culturing for 3 days. Here, "cell infection rate (%)" refers to the percentage (%) of the number of infected cells under each condition to the number of infected cells when infected in the absence of each compound, and "cell viability (%)" refers to the percentage (%) of the total number of viable cells under each condition to the total number of viable cells when infected in the absence of each compound.

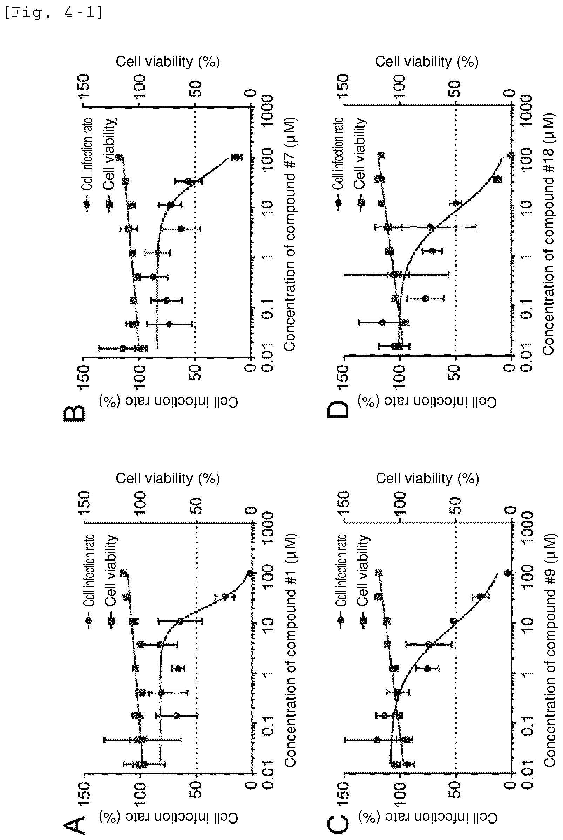

[Figure 4-1] Figure 4-1 is a graph showing the results of; infecting a Calu-3 cell line with SARS-CoV-2 (a prototype having D614G mutation) in the presence of 4 types of the present compounds (compound #1 [Figure 4-1A], compound #7 [Figure 4-1B], compound #9 [Figure 4-1C] and compound #18 [Figure 4-1D]); and analyzing the cell infection rate (%) and cell viability (%) after culturing for 3 days. Here, "cell infection rate (%)" refers to the percentage (%) of the number of infected cells under each condition to the number of infected cells when infected in the absence of each present compound, and "cell viability (%)" refers to the percentage (%) of the total number of viable cells under each condition to the total number of viable cells when infected in the absence of each present compound.

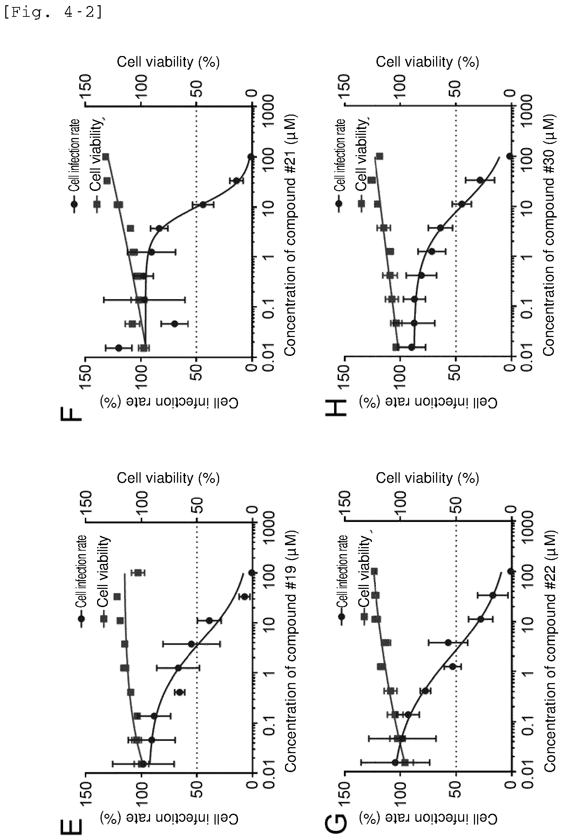

[Figure 4-2] Figure 4-2 is a graph showing the results of; infecting a Calu-3 cell line with SARS-CoV-2 (a prototype having D614G mutation) in the presence of 4 types of the present compounds (compound #19 [Figure 4-2E], compound #21 [Figure 4-2F], compound #22 [Figure 4-2G] and compound #30 [Figure 4-2H]); and analyzing the cell infection rate (%) and cell viability (%) after culturing for 3 days. Here, "cell infection rate (%)" refers to the percentage (%) of the number of infected cells under each condition to the number of infected cells when infected in the absence of each present compound, and "cell viability (%)" refers to the percentage (%) of the total number of viable cells under each condition to the total number of viable cells when infected in the absence of each present compound.

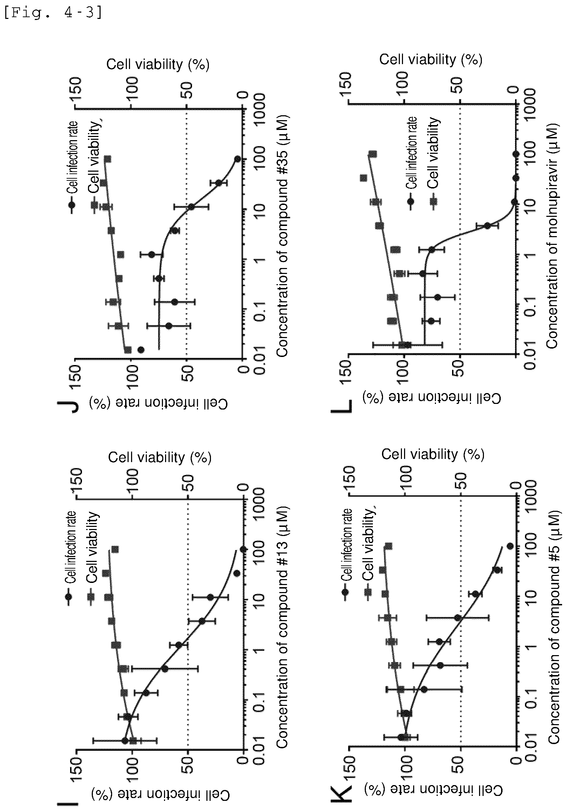

[Figure 4-3] Figure 4-3 is a graph showing the results of; infecting a Calu-3 cell line with SARS-CoV-2 (a prototype having D614G mutation) in the presence of 3 types of the present compounds (compound #13 [Figure 4-3I], compound #35 [Figure 4-3J] and compound #5 [Figure 4-3K]) or molnupiravir (Figure 4-3L); and analyzing the cell infection rate (%) and cell viability (%) after culturing for 3 days. Here, "cell infection rate (%)" refers to the percentage (%) of the number of infected cells under each condition to the number of infected cells when infected in the absence of each compound, and "cell viability (%)" refers to the percentage (%) of the total number of viable cells under each condition to the total number of viable cells when infected in the absence of each compound.

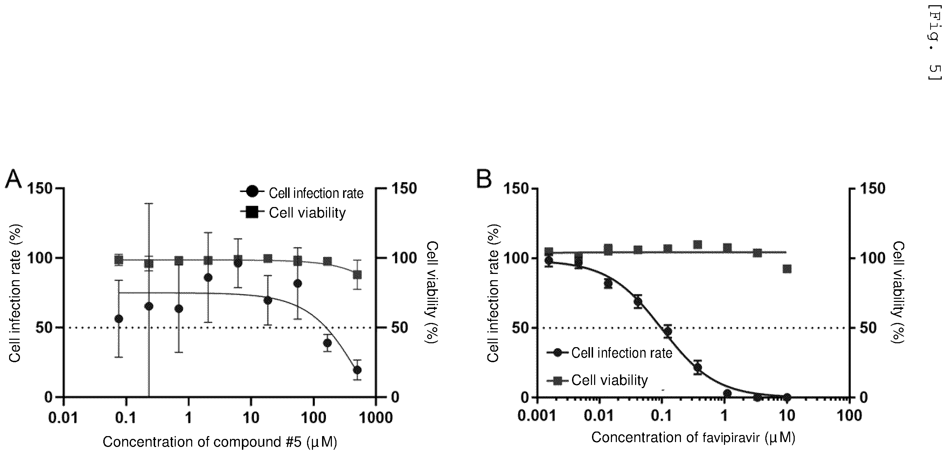

[Figure 5] Figure 5 is a graph showing the result of; infecting a Calu-3 cell line with type A influenza virus (subtype H1N1) in the presence of compound #5 (Figure 5A) or favipiravir (Figure 5B); and analyzing the cell infection rate (%) and cell viability (%) after culturing for 2 days. Here, "cell infection rate (%)" refers to the percentage (%) of the number of infected cells under each condition to the number of infected cells when infected in the absence of each compound, and "cell viability (%)" refers to the percentage (%) of the total number of viable cells under each condition to the total number of viable cells when infected in the absence of each compound.

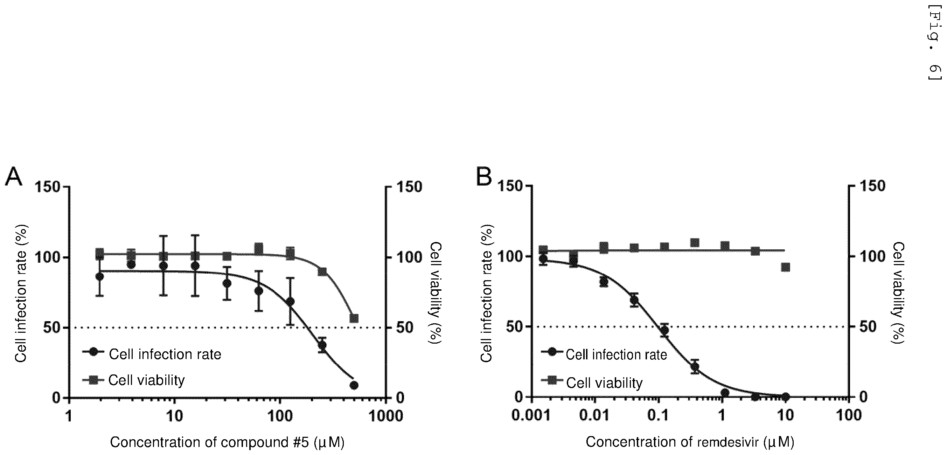

[Figure 6] Figure 6 is a graph showing the results of; infecting a human liver-derived cell line (Huh7 cell line) expressing Ebola virus NP/VP30/VP35/L protein with Ebola virus-derived trVLP (transcription and replication-competent virus-like particle) in the presence of compound #5 (Figure 6A) or remdesivir (Figure 6B); and analyzing the cell infection rate (%) and cell viability (%) after culturing for 2 days. Here, "cell infection rate (%)" refers to the percentage (%) of the number of infected cells under each condition to the number of infected cells when infected in the absence of each compound, and "cell viability (%) " refers to the percentage (%) of the total number of viable cells under each condition to the total number of viable cells when infected in the absence of each compound.

### Mode of Carrying Out the Invention

The antiviral agent of the present invention refers to an agent containing the present compound specified for the use "for an antiviral purpose" (hereinafter, sometimes referred to as "the subject antiviral agent"). In the subject antiviral agent, the present compound as an active ingredient may be used alone as a livestock feed, food and drink, or pharmaceutical (formulation), or may be used in the form of a composition (livestock feed composition, food and drink composition, or pharmaceutical composition) by further mixing it with an additive. Examples of the above-described food and drink include a health food (such as a functional food, a nutritional supplementary food, a health supplementary food, a nutritional fortified food, a nutritionally adjusted food or a supplement), and a health-prompting food (such as a food for specified health uses, a food with nutrient function claims or a food with functional claims).

As used herein, the term "antiviral" means preventing and/or ameliorating (treating) viral infections by such an action as inhibiting viral proliferation, inactivating a virus or reducing the susceptibility to a virus.

The target viruses of the subject antiviral agent are not particularly limited, and examples thereof include a DNA virus (such as a double-stranded DNA virus or a single-stranded DNA virus), an RNA virus (such as a double-stranded RNA virus, a single-stranded RNA (+) virus or a single-stranded RNA (-) virus). Specific examples of the target virus include influenza virus (a single-stranded RNA (-) virus), Ebola virus (a single-stranded RNA (-) virus), norovirus (a single-stranded RNA (+) virus), Japanese encephalitis virus (a single-stranded RNA (+) virus), rotavirus (a double-stranded RNA virus), rubella virus (a single-stranded RNA (+) virus), measles virus (a single-stranded RNA (+) virus), RS virus (a single-stranded RNA (-) virus), Filoviridae virus (a single-stranded RNA (-) virus), Arenaviridae virus (a single-stranded RNA (ambisense) virus), Bunyavirales virus (a single-stranded RNA (-) virus), herpesvirus (a double-stranded DNA virus), hepatitis A virus (a single-stranded RNA (+) virus), hepatitis B virus (a double-stranded DNA virus), hepatitis C virus (a single-stranded RNA (+) virus), hepatitis E virus (a single-stranded RNA (+) virus), smallpox virus (a double-stranded DNA virus), varicella zoster virus (a double-stranded DNA virus), adenovirus (a double-stranded DNA virus), foot-and-mouth disease virus (a single-stranded RNA (+) virus), dengue virus (a single-stranded RNA (+) virus), rabies virus (a single-stranded RNA (-) virus), human immunodeficiency virus (HIV) (a single-stranded RNA (+) virus), and coronavirus (a single-stranded RNA (+) virus). Suitable examples include coronavirus, influenza virus and Ebola virus, because the effects on these viruses have been demonstrated in the present Examples described below. The influenza virus includes types A, B and C influenza viruses and avian influenza virus, and various subtypes thereof. The coronavirus includes, in addition to a common coronavirus that causes the common cold, a novel coronavirus such as severe acute respiratory syndrome coronavirus (such as SARS, SARS-CoV-2 [such as that having a mutation in which the 614th aspartic acid in the spike protein is replaced with glycine [D614G mutation]], Middle East respiratory syndrome coronavirus (MERS), MERS-CoV), or a mutant thereof (such as a mutant [Delta mutant] of the B.1.617.2 strain of SARS-CoV-2, a mutant [Omicron mutant] of the B.1.1.529 strain of SARS-CoV-2 [such as Omicron mutant BA.1 strain or Omicron mutant BA.2 strain]).

The subject antiviral agent may be applied to any subject (that is, a mammal) in need of preventing or ameliorating (treating) viral infections. In the present specification, examples of the mammal include a human or a nonhuman mammal (such as a monkey, a mouse, a rat, a hamster, a ferret, a dog, a cat, or a livestock [such as a rabbit, a pig, a horse, a cow, a sheep, a goat or a deer]), with a human being a suitable example.

A detailed description of the compounds included in the present compound is given below.

In one embodiment of the present invention, R¹ in formula (I₀) represents a hydrogen atom. In other embodiment of the present invention, R¹ in formula (I₀) represents a benzoylmethyl group, the benzene ring of which is unsubstituted, or a benzoylmethyl group, the benzene ring of which is substituted with at least one group selected from the group consisting of an alkyl group having 1 to 7 carbon atoms, an alkoxy group having 1 to 7 carbon atoms, a fluorine atom and a chlorine atom. The term "a benzoylmethyl group, the benzene ring of which is substituted with at least one group selected from the group consisting of an alkyl group having 1 to 7 carbon atoms, an alkoxy group having 1 to 7 carbon atoms, a fluorine atom and a chlorine atom" means that one or more hydrogen atoms bonded to the carbon atoms constituting the benzene ring of the benzoylmethyl group are replaced with at least one group selected from the group consisting of an alkyl group having 1 to 7 carbon atoms, an alkoxy group having 1 to 7 carbon atoms, a fluorine atom and a chlorine atom. Therefore, the substituted benzene ring is a benzene ring in which 1 to 5 hydrogen atoms bonded to the carbon atoms constituting the benzene ring are replaced with an alkyl group having 1 to 7 carbon atoms, an alkoxy group having 1 to 7 carbon atoms, a fluorine atom or a chlorine atom. When the substituted benzene ring has two or more substituents, the substituents are the same or different. Examples thereof include a benzoylmethyl group having a benzene ring substituted with 1 to 5 alkyl groups having 1 to 7 carbon atoms, a benzene ring substituted with 1 to 5 alkoxy groups having 1 to 7 carbon atoms, a benzene ring substituted with 1 to 5 fluorine atoms, and a benzene ring substituted with 1 to 5 chlorine atoms. Other examples thereof include a benzoylmethyl group having a benzene ring substituted with a total of 2 to 5 substituents selected from an alkyl group having 1 to 4 carbon atoms, an alkoxy group having 1 to 4 carbon atoms, a fluorine atom and a chlorine atom. Examples of the alkyl group having 1 to 7 carbon atoms include a methyl group, an ethyl group, a propyl group, an isopropyl group, an n-butyl group, an isobutyl group, a sec-butyl group, a tert-butyl group, an n-pentyl group, a 1-methylbutyl group, a 2-methylbutyl group, a 3-methylbutyl group, a 1-ethylpropyl group, a 1,1-dimethylpropyl group, a 1,2-dimethylpropyl group, an n-hexyl group, a 1-methylpentyl group, a 2-methylpentyl group, a 3-methylpentyl group, a 4-methylpentyl group, a 1,1-dimethylbutyl group, a 1,2-dimethylbutyl group, a 1,3-dimethylbutyl group, a 2,2-dimethylbutyl group, a 2,3-dimethylbutyl group, a 3,3-dimethylbutyl group, a 1,1,2-trimethylpropyl group, a 1-ethylbutyl group, a 2-ethylbutyl group, a 1-ethyl-1-methylpropyl group, a 1-ethyl-2-methylpropyl group, a, an n-heptyl group, a 1-methylhexyl group, a 2-methylhexyl group, a 3-methylhexyl group, a 4-methylhexyl group, a 5-methylhexyl group, a 1-ethylpentyl group, a 2-ethylpentyl group, a 3-ethylpentyl group, a 4,4-dimethylpentyl group and a 1-propylbutyl group.

Example of the alkoxy group having 1 to 7 carbon atoms include a methoxy group, an ethoxy group, a propoxy group, an isopropoxy group, an n-butoxy group, an isobutoxy group, a sec-butoxy group, a tert-butoxy group, an n-pentoxy group, a 1-methylbutoxy group, a 2-methylbutoxy group, a 3-methylbutoxy group, a 1-ethylpropoxy group, a 1,1-dimethylpropoxy group, a 1,2-dimethylpropoxy group, a 2,2-dimethylpropoxy group, an n-hexyloxy group, a 1-methylpentyloxy group, a 2-methylpentyloxy group, a 3-methylpentyloxy group, a 4-methylpentyloxy group, a 1,1-dimethylbutoxy group, a 1,2-dimethylbutoxy group, a 1,3-dimethylbutoxy group, a 2,2-dimethylbutoxy group, a 2,3-dimethylbutoxy group, a 3,3-dimethylbutoxy group, a 1,1,2-trimethylpropoxy group, a 1-ethylbutoxy group, a 2-ethylbutoxy group, a 1-ethyl-1-methylpropoxy group, a 1-ethyl-2-methylpropoxy group, an n-heptyloxy group, a 1-methylhexyloxy group, a 2-methylhexyloxy group, a 3-methylhexyloxy group, a 4-methylhexyloxy group, a 5-methylhexyloxy group, a 1-ethylpentyloxy group, a 2-ethylpentyloxy group, a 3-ethylpentyloxy group, a 4,4-dimethylpentyloxy group and a 1-propylbutoxy group.

In other embodiment of the present invention, R¹ in formula (I₀) represents a linear or branched alkyl group, having 4 to 6 carbon atoms, unsubstituted or substituted with a fluorine atom. Examples of the linear or branched alkyl group, having 4 to 6 carbon atoms, unsubstituted or substituted with a fluorine atom include an n-butyl group, an isobutyl group, a sec-butyl group, a tert-butyl group, an n-pentyl group, a 1-methylbutyl group, a 2-methylbutyl group, a 3-methylbutyl group, a 1-ethylpropyl group, a 1,1-dimethylpropyl group, a 1,2-dimethylpropyl group, a 2,2-dimethylpropyl group, an n-hexyl group, a 1-methylpentyl group, a 2-methylpentyl group, a 3-methylpentyl group, a 4-methylpentyl group, a 1,1-dimethylbutyl group, a 1,2-dimethylbutyl group, a 1,3-dimethylbutyl group, a 2,2-dimethylbutyl group, a 2,3-dimethylbutyl group, a 3,3-dimethylbutyl group, a 1,1,2-trimethylpropyl group, a 1-ethylbutyl group, a 2-ethylbutyl group, a 1-ethyl-1-methylpropyl group, a 1-ethyl-2-methylpropyl group, and fluorinated forms thereof. It is preferably a 1-ethylbutyl group, a 2-ethylbutyl group, a 1-methylpentyl group, a 2-methylpentyl group, a 3-methylpentyl group, a 4-methylpentyl group, a 5-methylpentyl group, a 3,3,4,4,4-pentafluorobutyl group, a 4,4,5,5,5-pentafluoropentyl group or a 5,5,6,6,6-pentafluorohexyl group; more preferably a 2-ethylbutyl group, a 2-methylpentyl group, a 3-methylpentyl group or a 4,4,5,5,5-pentafluoropentyl group; and most preferably a 4,4,5,5,5-pentafluoropentyl group.

In other embodiment of the present invention, R¹ in formula (I₀) represents an alkyl group, having 1 to 6 carbon atoms, substituted with a group of formula (A) (wherein * indicates the bonding position) and optionally having an oxygen atom. Examples of the alkyl group having 1 to 6 carbon atoms include a methyl group, an ethyl group, a propyl group, an isopropyl group, an n-butyl group, an isobutyl group, a sec-butyl group, a tert-butyl group, an n-pentyl group, a 1-methylbutyl group, a 2-methylbutyl group, a 3-methylbutyl group, a 1-ethylpropyl group, a 1,1-dimethylpropyl group, a 1,2-dimethylpropyl group and an n-hexyl group. The term "alkyl group, having 1 to 6 carbon atoms, having an oxygen atom" means an alkyl group having 1 to 6 carbon atoms in which an oxygen atom is ether-bonded in the skeleton or at the end thereof. Examples thereof include (A)-methylene-O-methylene-, (A)-methylene-O-ethylene-, (A)-ethylene-O-ethylene- and (A)-propylene-O-.

In other embodiment of the present invention, R¹ in formula (I₀) represents a methyl group or an ethyl group, substituted with a phenyl group or a cyclopentyl group, the phenyl group being optionally further substituted with one or more phenyl groups. The methyl group or the ethyl group substituted with a phenyl group or a cyclopentyl group is a benzyl group, a 2-phenethyl group, a cyclopentylmethyl group or a 2-cyclopentylethyl group. Examples of the benzyl group or 2-phenethyl group substituted with one or more phenyl groups include a 3-phenylbenzyl group, a 4-phenylbenzyl group, a 3,5-diphenylbenzyl group, a 2-(1,1'-biphenyl-3-yl)-ethyl group, a 2-(1,1'-biphenyl-4-yl)-ethyl group and a 2-(3,5-diphenylphenyl)-ethyl group. Suitable examples of R¹ in formula (I) include a 2-phenethyl group, a cyclopentylmethyl group, a 2-cyclopentylethyl group and a 2-(1,1'-biphenyl-3-yl)-ethyl group.

Z¹, Z², Z³ and Z⁴ in formula (I₀) are the same or different and each represent a hydrogen atom, a halogen atom, a C1-C6 alkyl group, a C2-C6 alkenyl group, a C2-C6 alkynyl group or a group of OR⁸ wherein R⁸ represents a C1-C7 alkyl group, a C2-C6 alkenyl group or a C2-C6 alkynyl group. Z⁵ can represent a hydrogen atom or a C1-C10 alkyl group. Examples of the halogen atom include a fluorine atom, a chlorine atom, a bromine atom or an iodine atom. Examples of the C1-C6 alkyl group, C1-C7 alkyl group and C1-C10 alkyl group include a methyl group, an ethyl group, a propyl group, an isopropyl group, an n-butyl group, an isobutyl group, a sec-butyl group, a tert-butyl group, an n-pentyl group, a 1-methylbutyl group, a 2-methylbutyl group, a 3-methylbutyl group, a 1-ethylpropyl group, a 1,1-dimethylpropyl group, a 1,2-dimethylpropyl group, a 2,2-dimethylpropyl group, an n-hexyl group, a 1-methylpentyl group, a 2-methylpentyl group, a 3-methylpentyl group, a 4-methylpentyl group, a 1,1-dimethylbutyl group, a 1,2-dimethylbutyl group, a 1,3-dimethylbutyl group, a 2,2-dimethylbutyl group, a 2,3-dimethylbutyl group, a 3,3-dimethylbutyl group, a 1,1,2-trimethylpropyl group, a 1-ethylbutyl group, a 2-ethylbutyl group, a 1-ethyl-1-methylpropyl group and a 1-ethyl-2-methylpropyl group. Examples of the C2-C6 alkenyl group include an ethenyl group (vinyl group), a 1-propenyl group, a 2-propenyl group (allyl group), a 1-butenyl group, a 2-butenyl group, a 3-butenyl group, an isobutenyl group, a 1-pentenyl group, a 2-pentenyl group, a 3-pentenyl group, a 4-pentenyl group, a 1-hexenyl group, a 2-hexenyl group, a 3-hexenyl group, a 4-hexenyl group and a 5-hexenyl group. Examples of the C2-C6 alkynyl group include an ethynyl group, a 1-propynyl group, a 2-propynyl group (propargyl group), a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, a 1,1-dimethyl-2-butynyl group, an n-heptyl group, an n-octyl group and an n-decyl group. Examples of the C1-C7 alkoxy group (the group of OR⁸ wherein R⁸ represents a C1-C7 alkyl group) include a methoxy group, an ethoxy group, a propoxy group, an isopropoxy group, an n-butoxy group, an isobutoxy group, a sec-butoxy group, a tert-butoxy group, an n-pentoxy group, a 1-methylbutoxy group, a 2-methylbutoxy group, a 3-methylbutoxy group, a 1-ethylpropoxy group, a 1,1-dimethylpropoxy group, a 1,2-dimethylpropoxy group, a 2,2-dimethylpropoxy group, an n-hexyloxy group, a 1-methylpentyloxy group, a 2-methylpentyloxy group, a 3-methylpentyloxy group, a 4-methylpentyloxy group, a 1,1-dimethylbutoxy group, a 1,2-dimethylbutoxy group, a 1,3-dimethylbutoxy group, a 2,2-dimethylbutoxy group, a 2,3-dimethylbutoxy group, a 3,3-dimethylbutoxy group, a 1,1,2-trimethylpropoxy group, a 1-ethylbutoxy group, a 2-ethylbutoxy group, a 1-ethyl-1-methylpropoxy group, a 1-ethyl-2-methylpropoxy group, an n-heptyloxy group, a 1-methylhexyloxy group, a 2-methylhexyloxy group, a 3-methylhexyloxy group, a 4-methylhexyloxy group, a 5-methylhexyloxy group, a 1-ethylpentyloxy group, a 2-ethylpentyloxy group, a 3-ethylpentyloxy group, a 4,4-dimerpentyloxy group and a 1-propylbutoxy group. Preferably, Z¹, Z², Z³ and Z⁴ are the same or different and each represent hydrogen, an ethoxy group, fluorine or chlorine.

R⁴ and R⁵ in formula (I₀) are the same or different and each represents a substituted or unsubstituted alkyl group having 1 to 4 carbon atoms. Examples of the substituted or unsubstituted alkyl group having 1 to 4 carbon atoms include a methyl group, an ethyl group, a propyl group, an isopropyl group, an n-butyl group, an isobutyl group, a sec-butyl group, a tert-butyl group, pyrrolidine in which R⁴ and R⁵ form together with nitrogen, and those substituted with a methoxy group, a phenyl group, fluorine and chlorine. It is preferably a methyl group, a monochloromethyl group, an ethyl group, a 2-methoxyethyl group, a 2,2,2-trichloroethyl group, a 1-phenylethyl group, a 2-phenylethyl group, a methoxyethyl group, an isopropyl group, a hexafluoroisopropyl group or pyrrolidine, and more preferably a methyl group or an ethyl group.

Examples of the implementation of formula (I₀) include a compound of formula (I) and preferably a compound of formula (1). 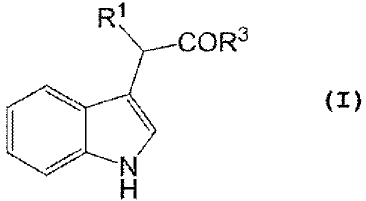 [wherein R¹ and R³ have the same meanings as defined in [1] above] 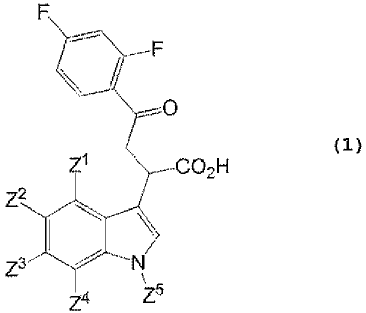 [wherein Z¹, Z², Z³, Z⁴ and Z⁵ have the same meanings as defined in [1] above]

In the compound of formula (1), Z¹, Z², Z³ and Z⁴ are the same or different and each represent a hydrogen atom, a halogen atom, a C1-C6 alkyl group, a C2-C6 alkenyl group, a C2-C6 alkynyl group or a group of OR⁸ wherein R⁸ represents a C1-C7 alkyl group, a C2-C6 alkenyl group or a C2-C6 alkynyl group; and Z⁵ represents a hydrogen atom or a C1-C10 alkyl group.

Examples of the halogen atom in formula (1) include a fluorine atom, a chlorine atom, a bromine atom and an iodine atom.

The C1-C6 alkyl group in formula (1) means a linear or branched alkyl group, having 1 to 6 carbon atoms, optionally having a substituent, and specific examples thereof include a methyl group, an ethyl group, an n-propyl group, an isopropyl group, an n-butyl group, an isobutyl group, a sec-butyl group, a tert-butyl group, an n-pentyl group, an isopentyl group, a neopentyl group and an n-hexyl group.

Examples of the substituent in the "optionally having a substituent" include a halogen atom, a hydroxyl group, a carboxy group, an alkyl group having 1 to 6 carbon atoms, an alkenyl group having 2 to 6 carbon atoms, an alkynyl group having 2 to 6 carbon atoms and a C6-C10 aryl group. The alkyl group having 1 to 6 carbon atoms, the alkenyl group having 2 to 6 carbon atoms and the alkynyl group having 2 to 6 carbon atoms as described above are the same as the alkyl group having 1 to 6 carbon atoms, the alkenyl group having 2 to 6 carbon atoms and the alkynyl group having 2 to 6 carbon atoms in formula (1). Examples of the C6-C10 aryl group include a phenyl group and a naphthyl group.

The C2-C6 alkenyl group in formula (1) means a linear or branched alkenyl group, having 2 to 6 carbon atoms, optionally having a substituent, and specific examples thereof include a vinyl group, a 1-propenyl group, a 2-propenyl group, a 1-butenyl group, a 2-butenyl group, a 3-butenyl group, a 1,3-butenyl group, a 1-pentenyl group and a 1-hexenyl group.

The C2-C6 alkynyl group in formula (1) means a linear or branched alkynyl group, having 2 to 6 carbon atoms, optionally having a substituent, and specific examples thereof include an ethynyl group, a 1-propynyl group, a 1-butynyl group, a 1-pentynyl group and a 1-hexynyl group.

Z¹, Z², Z³ and Z⁴ are preferably a hydrogen atom, a halogen atom, a C1-C6 alkyl group or a group of OR⁸, and more preferably a hydrogen atom, a fluorine atom, a chlorine atom, a C1-C3 alkyl group such as a methyl group, an ethyl group, an n-propyl group or an isopropyl group, or a group of OR⁸.

Z⁵ is preferably a hydrogen atom or a C1-C3 alkyl group, and more preferably a hydrogen atom or a methyl group.

R⁸ is preferably a C1-C6 alkyl group, and more preferably a C1-C3 alkyl group such as a methyl group, an ethyl group, an n-propyl group or an isopropyl group, or a benzyl group.

Among the compound of formula (1), the compound of the following formula (2), formula (3), formula (4), formula (5) or formula (6) or a salt thereof is preferred.

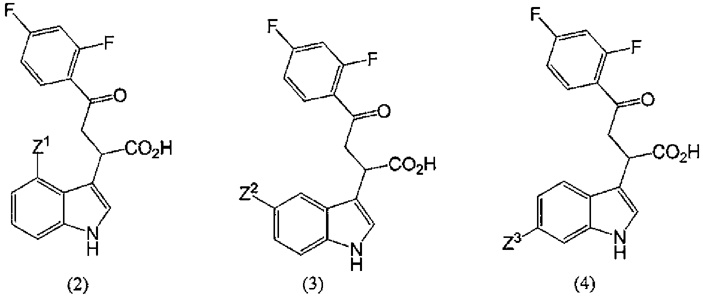

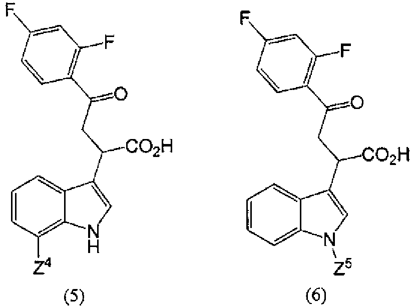

In above formula (2), formula (3), formula (4), formula (5) and formula (6), Z¹, Z², Z³, Z⁴ and Z⁵ are defined in the same manner as Z¹, Z², Z³, Z⁴ and Z⁵ in formula (1).

Specific examples of the compound of formula (1) include the compounds described below:

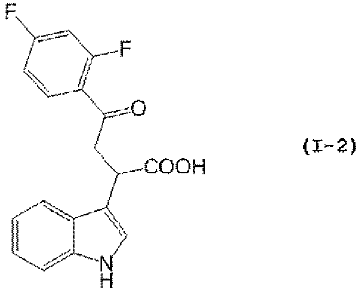

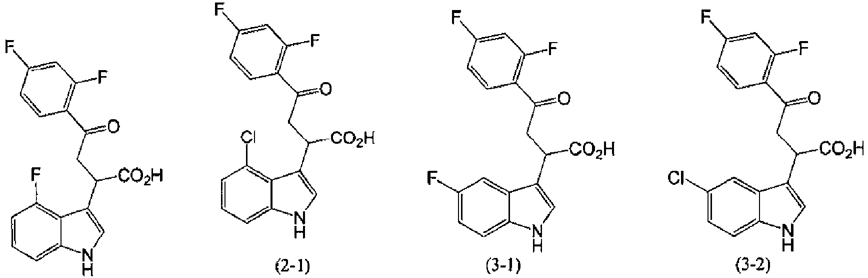

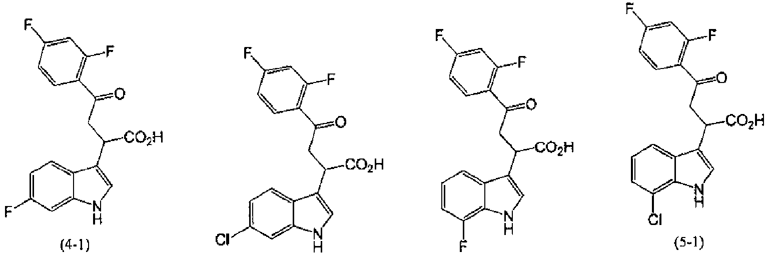

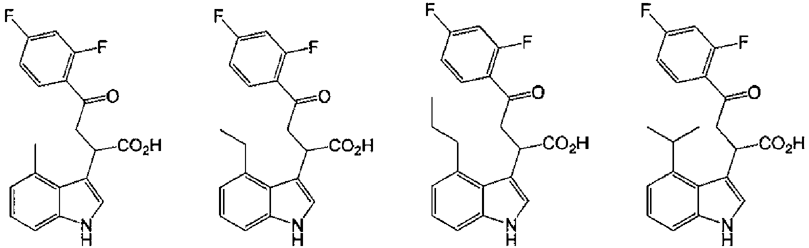

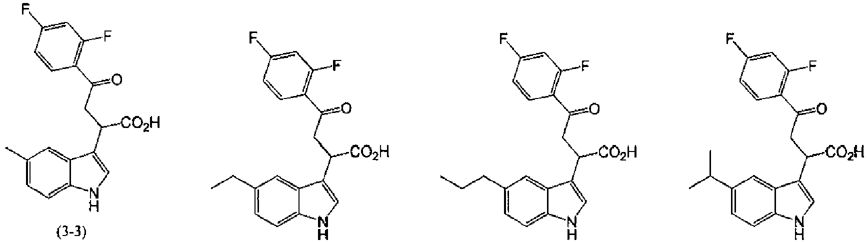

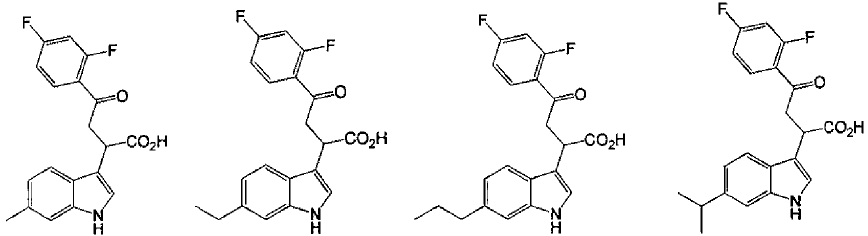

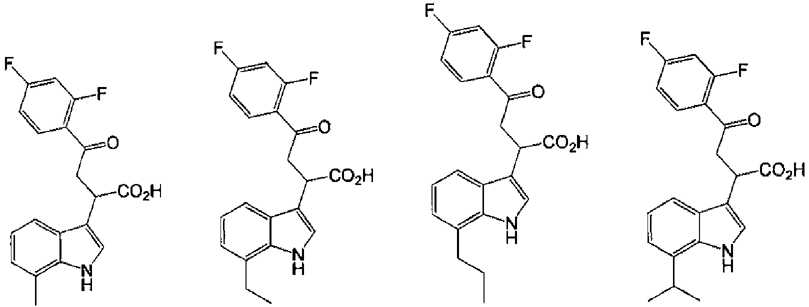

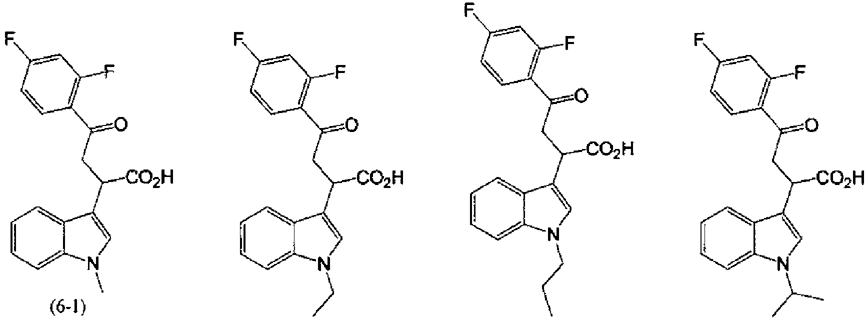

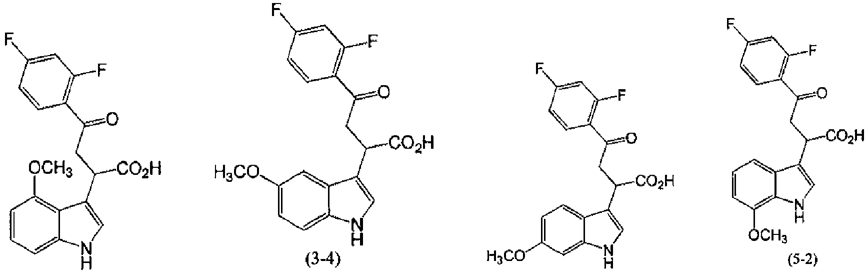

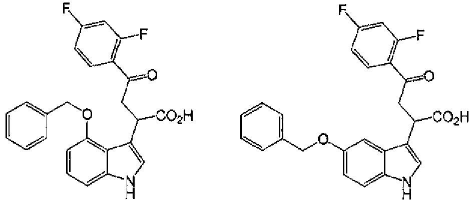

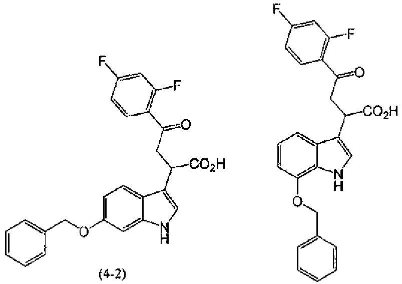

Among the above compounds, the following compounds are preferred.

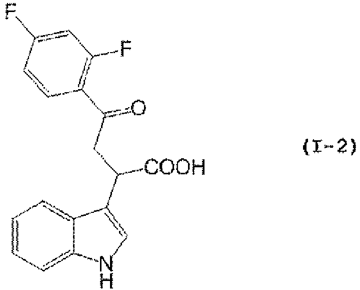

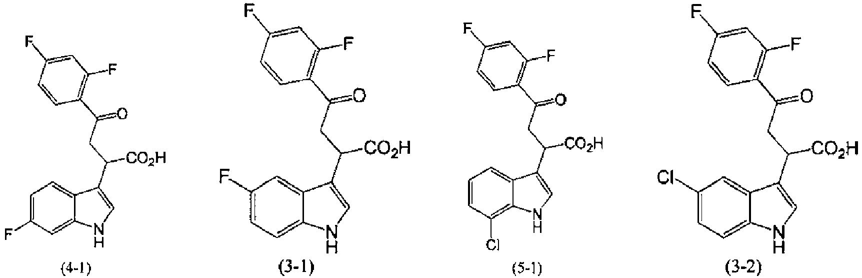

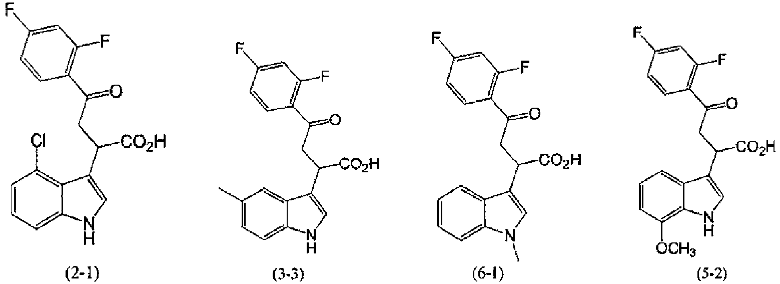

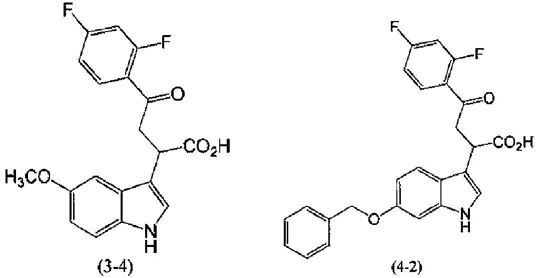

The compound of formula (I₀) wherein R¹ represents a 2,4-difluorobenzoylmethyl group, Z¹, Z², Z³, Z⁴ and Z⁵ each represent hydrogen and R³ represents OH, represents a compound of the following formula (I-2) (compound #5 described below in Examples);
the compound of formula (I₀) wherein R¹ represents a benzoylmethyl group, Z¹ represents chlorine, Z², Z³, Z⁴ and Z⁵ each represent hydrogen and R³ represents OH, represents a compound of the following formula (I-3) (compound #1 described below in Examples); the compound of formula (I₀) wherein R¹ represents a benzoylmethyl group, Z² represents an ethoxy group, Z¹, Z³, Z⁴ and Z⁵ each represent hydrogen and R³ represents OH, represents a compound of the following formula (I-4) (compound #7 described below in Examples);
the compound of formula (I₀) wherein R¹ represents a (1-acetyl-4-piperidinyl)methyl group, Z¹, Z², Z³, Z⁴ and Z⁵ each represent hydrogen and R³ represents OH, represents a compound of the following formula (I-5) (compound #9 described below in Examples);
the compound of formula (I₀) wherein R¹ represents a 3-methyl-1-pentyl group, Z¹, Z², Z³, Z⁴ and Z⁵ each represent hydrogen and R³ represents OH, represents a compound of the following formula (I-6) (compound #18 described below in Examples);
the compound of formula (I₀) wherein R¹ represents a 2-methyl-1-pentyl group, Z¹, Z², Z³, Z⁴ and Z⁵ each represent hydrogen and R³ represents OH, represents a compound of the following formula (I-7) (compound #19 described below in Examples);
the compound of formula (I₀) wherein R¹ represents a 4,4,5,5,5-pentafluoro-1-pentyl group, Z¹, Z², Z³, Z⁴ and Z⁵ each represent hydrogen and R³ represents OH, represents a compound of the following formula (I-8) (compound #21 described below in Examples);
the compound of formula (I₀) wherein R¹ represents a 2-(1,1'-biphenyl-3-yl) -1-ethyl group, Z¹, Z², Z³, Z⁴ and Z⁵ each represent hydrogen and R³ represents OH, represents a compound of the following formula (I-9) (compound #22 described below in Examples); and
the compound of formula (I₀) wherein R¹ represents a hydrogen atom, Z¹, Z², Z³ and Z⁴ each represent hydrogen, Z⁵ represents an n-hexyl group and R³ represents OH, represents a compound of the following formula (I-10) (compound #30 described below in Examples).

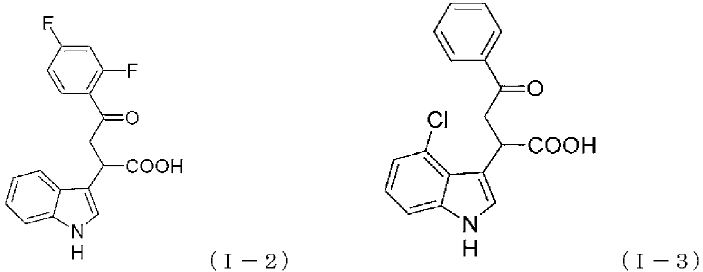

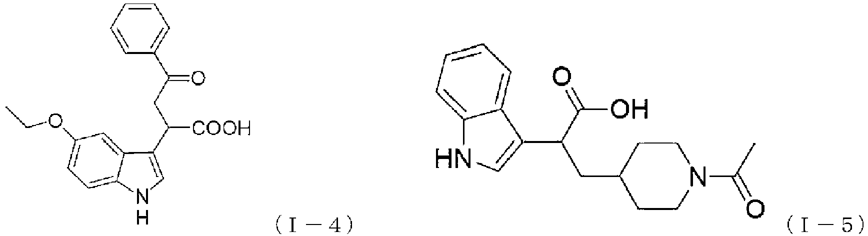

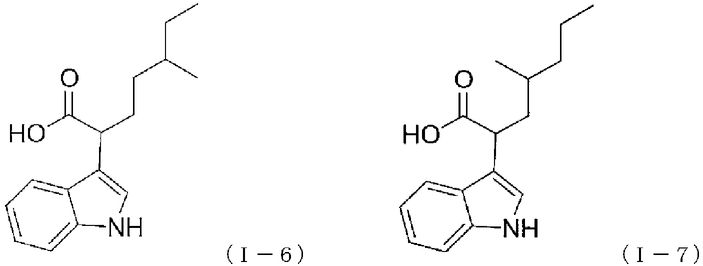

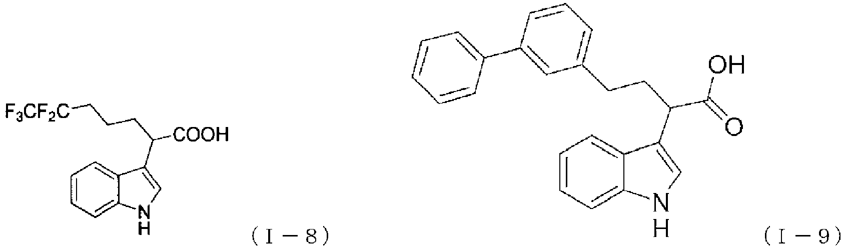

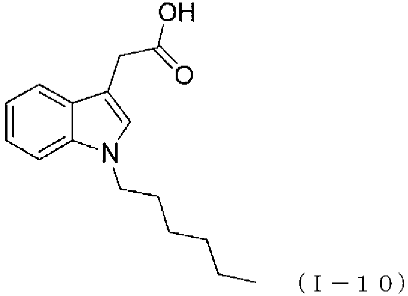

X in formula (II) represents an alkylene group, having 4 to 6 carbon atoms, optionally having an oxygen atom. Here, the alkylene group having 4 to 6 carbon atoms means butylene-(CH₂)₄-, pentylene- (CH₂)₅- or hexylene- (CH₂)₆-; and the alkylene group, having 4 to 6 carbon atoms, having an oxygen atom means an alkyl group having 4 to 6 carbon atoms in which an oxygen atom is ether-bonded in the skeleton or at the end thereof. Examples thereof include a methylene-O-propylene group, an ethylene-O-ethylene group and a propylene-O-methylene group, and it is preferably butylene, hexylene or an ethylene-O-ethylene group.

R⁴ and R⁵ in formula (II) are the same or different and each represent a substituted or unsubstituted alkyl group having 1 to 4 carbon atoms. Examples of the substituted or unsubstituted alkyl group having 1 to 4 carbon atoms include a methyl group, an ethyl group, a propyl group, an isopropyl group, an n-butyl group, an isobutyl group, a sec-butyl group, a tert-butyl group, pyrrolidine in which R⁴ and R⁵ form together with nitrogen, and those substituted with a methoxy group, a phenyl group, fluorine and chlorine. It is preferably a methyl group, a monochloromethyl group, an ethyl group, a 2,2,2-trichloromethyl group, a 1-phenylethyl group, a 2-phenylethyl group, a methoxyethyl group, an isopropyl group, a hexafluoroisopropyl group or pyrrolidine, and more preferably a methyl group or an ethyl group.

The compound of formula (II) wherein X represents a hexylene group, R⁶ represents a methyl group and R³ represents OH, represents a compound of the following formula (II-2) (compound #13 described below in Examples).

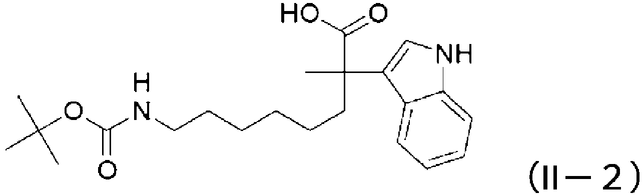

R⁷ in formula (III) is an alkyl group having 1 to 10 carbon atoms or a benzyl group. Examples of the linear or branched alkyl group having 1 to 10 carbon atoms include a methyl group, an ethyl group, a propyl group, an isopropyl group, an n-butyl group, an isobutyl group, a sec-butyl group, a tert-butyl group, an n-pentyl group, a 1-methylbutyl group, a 2-methylbutyl group, a 3-methylbutyl group, a 1-ethylpropyl group, a 1,1-dimethylpropyl group, a 1,2-dimethylpropyl group, a 2,2-dimethylpropyl group, an n-heptyl group, an n-octyl group and an n-decyl group. The benzene ring of the benzyl group is optionally substituted with one or more of an alkyl group having 1 to 3 carbon atoms or an alkoxy group having 1 to 3 carbon atoms. Examples of the alkyl group having 1 to 3 carbon atoms include a methyl group, an ethyl group, an n-propyl group and an isopropyl group; and examples of the alkoxy groups having 1 to 3 carbon atoms include a methoxy group, an ethoxy group, an n-propoxy group and an isopropoxy group. R⁷ in formula (III) is preferably a methyl group, an ethyl group, a propyl group, an n-butyl group, an n-pentyl group or a 3,5-dimethoxybenzyl group, and more preferably 3,5-dimethoxybenzyl group.

R⁴ and R⁵ in formula (III) are the same or different and each represents a substituted or unsubstituted alkyl group having 1 to 4 carbon atoms. Examples of the substituted or unsubstituted alkyl group having 1 to 4 carbon atoms include a methyl group, an ethyl group, a propyl group, an isopropyl group, an n-butyl group, an isobutyl group, a sec-butyl group, a tert-butyl group, pyrrolidine in which R⁴ and R⁵ form together with nitrogen, and those substituted with a methoxy group, a phenyl group, fluorine and chlorine. It is preferably a methyl group, a monochloromethyl group, an ethyl group, a 2,2,2-trichloromethyl group, a 1-phenylethyl group, a 2-phenylethyl group, a methoxyethyl group, an isopropyl group, a hexafluoroisopropyl group or pyrrolidine, and more preferably a methyl group or an ethyl group.

The compound of formula (III) wherein A represents indole, R⁷ represents a 3,5-dimethoxybenzyl group and R³ represents OH, represents a compound of the following formula (III-2) (compound #35 described below in Examples).

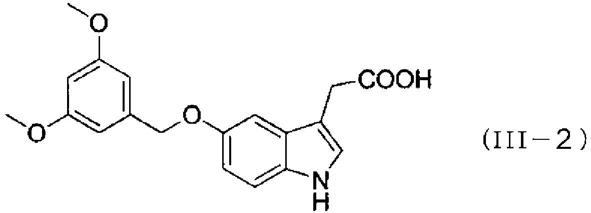

When the present compound has an asymmetric carbon atom and an asymmetric center associated with the axial chirality, it comprises all possible optical isomers, and the optical isomers can be used in any ratio. For example, a certain optically active compound can be used as an enantiomer, a racemate or a mixture of enantiomers in any ratio, and when multiple asymmetric centers are present, a mixture of diastereomers in any ratio may be used.

The pharmaceutically acceptable salt of the present compound comprises a metal salt produced from aluminum, calcium, lithium, magnesium, potassium, sodium and zinc, and an organic salt produced from N,N'-dibenzylethylenediamine, chloroprocaine, choline, diethanolamine, ethylenediamine, N-methylglucamine, lysine or procaine.

The method for synthesizing the present compound to be used can be a method disclosed in Patent Document 3, International Publication No. WO 2019/235455, Japanese unexamined Patent Application Publication NO. 2023-24388, or the like, but is not limited to these methods. The present compound can be also synthesized by using a generally known synthesis method or and be available from Sigma-Aldrich Co. LLC, Tokyo Chemical Industry Co., Ltd., Wako Pure Chemical Industries, Ltd., Kanto Chemical Co. Inc., or the like.

The present compound is preferably 11 compounds (compound #5, compound #1, compound #7, compound #9, compound #13, compound #18, compound #19, compound #21, compound #22, compound #30 and compound #35) the effects of which are specifically shown in Examples in the specification of the present application. Compound #5 may be a racemate or an enantiomer (R-isomer or S-isomer), but the R-isomer of compound #5 can be suitably exemplified because its effect has been demonstrated in Examples described below.

Examples of the additive for the subject antiviral agent include a formulation component such as a pharmaceutically acceptable conventional carrier, binder, stabilizer, excipient, diluent, pH buffer, disintegrant, isotonic agent, additive, coating agent, solubilizer, lubricating agent, glidant, dissolution aid, lubricant, flavoring agent, sweetener, solvent, gelling agent or nutrient. Specific examples of such a formulation component include water, physiological saline, animal fat and oil, a vegetable oil, lactose, starch, gelatin, crystalline cellulose, gum, talc, magnesium stearate, hydroxypropyl cellulose, polyalkylene glycol, polyvinyl alcohol and glycerin.

The administration form of the subject antiviral agent includes oral administration in which the administration is carried out in such a dosage form as a powder, a granule, a tablet, a capsule, a syrup or a suspension; injection in which the administration is carried out in such a dosage form as a solution, an emulsion or a suspension; and parenteral administration in which intranarial administration is carried out in the dosage form of a spray.

The dosage of the subject antiviral agent is appropriately determined depending on the age, body weight, sex, symptoms, drug sensitivity, or the like, and is, for example, in the range of 1 µg to 200 mg/kg (body weight) /day. The subject antiviral agent is administered once or multiple times (for example, 2 to 4 times) per day, and the dosage may be adjusted depending on the improvement of symptoms.

The subject antiviral agent may comprise an antiviral component in addition to the present compound, but since the present compound alone exerts an excellent antiviral effect, it preferably comprises, in addition to the present compound, no antiviral component (such as a compound [such as an antibiotic], a protein [such as an antibody], DNA or RNA).

### Examples

The present invention will be described in more detail with reference to Examples below, but the technical scope of the present invention is not limited thereto.

### Example 1

### 1. Present compound

11 present compounds (compound #5, compound #1, compound #7, compound #9, compound #18, compound #19, compound #21, compound #22, compound #30, compound #13 and compound #35) were synthesized according to any one of methods described in Patent Document 3 and International Publication No. WO 2019/235455. The R-isomer of compound #5 was separated from compound #5 (a racemate) according to the method described in Japanese unexamined Patent Application Publication No. 2023-24388.

### Example 2

### 2. Confirmation that compound #5 has an antiviral action

In order to confirm that compound #5 has an antiviral action, an experiment was carried out according to the method described under the item "2-1. Method" below.

### 2-1. Method

[1] A human bronchial epithelial adenocarcinoma cell line (Calu-3 cell line) (distributed by the National Institute of Infectious Diseases) was seeded in a 96-well plate and cultured overnight at 37°C.
[2] The Calu-3 cell line was cultured (pretreated) for 1 to 2 hours at 37°C in the presence of 2 test substances (compound #5 or molnupiravir [manufactured by Selleck Biotechnology]) at various concentrations (0.390625 µM, 0.78125 µM, 1.5625 µM, 3.125 µM, 6.25 µM, 12.5 µM, 25 µM, 50 µM and 100 µM).
[3] Thereafter, the Calu-3 cell line was infected with 3 types of SARS-CoV-2 (a prototype having D614G mutation [JPN/NGS/SC-1/2020, independently isolated from clinical specimens], Omicron mutant BA.1 strain [hCoV-19/Japan/TY38-873/2021, obtained from the National Institute of Infectious Diseases], or Omicron mutant BA.2 strain [hCoV-19/Japan/TY40-385/2022, obtained from the National Institute of Infectious Diseases]), in the presence of the 2 test substances at an MOI (multiplicity of infection) of 0.1 for 3 days.
[4] The infected Calu-3 cell line was fixed overnight in a solution containing 4% paraformaldehyde (PFA).
[5] It was subjected to immunostaining using a monoclonal antibody against a SARS-CoV-2 N protein (bsm-41414M, manufactured by Bioss Antibodies) according to any conventional method to detect a SARS-CoV-2 N protein positive cell (that is, a cell infected with various SARS-CoV-2 viruses; hereinafter, referred to as an "infected cell").
[6] All cell nuclei were stained using Hoechst 33342 (manufactured by Thermo Fisher Scientific).
[7] A Cytation 5 cell imaging multimode plate reader (manufactured by BioTek) was used to acquire fluorescence images, and a CellProfiler image analysis software (manufactured by Broad Institute) was used to calculate the number of infected cells and total number of cells.
[8] GraphPad Prism (manufactured by GraphPad Software) was used to create graphs showing the relationships between the concentrations of the above 2 test substances, and the cell infection rate (%) and cell viability (%) (Figures 1 and 2), and to calculate IC50 (that is, the concentration of the test substance required to reduce the number of infected cells to 50% of the number of infected cells when infected in the absence of the test substance) (Table 1).

2-2. Results

The results shows that when the Calu-3 cell line was infected with 3 types of SARS-CoV-2 (a prototype having D614G mutation, Omicron mutant BA.1 strain, or Omicron mutant BA.2 strain) in the presence of compound #5 (Figure 1), the cell infection rate decreased in a concentration-dependent manner, similarly to when the Calu-3 cell line was infected with 3 types of SARS-CoV-2 in the presence of the existing antiviral drug, molnupiravir (Figure 2). The results of calculating IC50 confirmed that compound #5 exerts the antiviral effect comparable to that of molnupiravir (Table 1).

**[Table 1]**

| SARS-CoV-2 | IC50(µM) | |
|---|---|---|
| | Compound #5 | Molnupiravir |
| Prototype having D614G mutation | 1.2 | 4.2 |
| Omicron mutant BA.1 strain | 16.7 | 4.0 |
| Omicron mutant BA.2 strain | 3.6 | 2.8 |

### Example 3

### 3. Confirmation that specific enantiomer of compound #5 exerts an antiviral action at lower concentration

In order to analyze whether there is a difference in the concentration at which the antiviral effect is exerted among the racemate, R-isomer and S-isomer of compound #5, an experiment was carried out according to the method described under the item "3-1. Method" below.

### 3-1. Method

[1] Calu-3 cell line was inoculated in a 96-well plate and cultured overnight at 37°C.
[2] The Calu-3 cell line was cultured (pretreated) for 1 to 2 hours at 37°C in the presence of 3 types of compound #5 (an R-isomer, an S-isomer and a racemate) or molnupiravir at various concentrations (0.015242 µM, 0.045725 µM, 0.137174 µM, 0.411523 µM, 1.234568 µM, 3.703704 µM, 11.11111 µM, 33.33333 µM and 100 µM).
[3] Thereafter, the Calu-3 cell line was infected with SARS-CoV-2 (a prototype having D614G mutation) in the presence of the above 3 types of compound #5 or molnupiravir at MOI of 0.1 for 3 days.
[4] The infected Calu-3 cell line was fixed overnight in a solution containing 4% PFA.
[5] It was subjected to immunostaining using a monoclonal antibody against a SARS-CoV-2 N protein (bsm-41414M, manufactured by Bioss Antibodies) according to any conventional method to detect an infected cell.
[6] All cell nuclei were stained using Hoechst 33342 (manufactured by Thermo Fisher Scientific).
[7] A Cytation 5 cell imaging multimode plate reader (manufactured by BioTek) was used to acquire fluorescence images, and a CellProfiler image analysis software (manufactured by Broad Institute) was used to calculate the number of infected cells and total number of cells.
[8] GraphPad Prism (manufactured by GraphPad Software) was used to create graphs showing the relationships between the concentrations of the above 3 types of compound #5 or molnupiravir, and the cell infection rate (%) and cell viability (%) (Figures 3), and to calculate IC50 (Table 2).

### 3-2. Results

The results show that when the Calu-3 cell line was infected with SARS-CoV-2 (a prototype having D614G mutation) in the presence of the R-isomer of compound #5, the IC50 value was lower than when the Calu-3 cell line was infected with the above SARS-CoV-2 in the presence of the S-isomer and racemate of compound #5 (Figure 3 and Table 2). These results indicate that the R-isomer of compound #5 exerts an antiviral effect at a lower concentration than the S-isomer and racemate of compound #5.

**[Table 2]**

| Test substance | IC50 (µM) |
|---|---|
| R-isomer of compound #5 | 1.0 |
| S-isomer of compound #5 | 5.7 |
| Racemate of compound #5 | 2.0 |
| Molnupiravir | 1.0 |

### Example 4

### 4. Confirmation that present compounds other than compound #5 also have an antiviral action

In order to confirm that the present compounds other than compound #5 also have an antiviral action, an experiment was conducted according to the method described under the item "4-1. Method" below.

### 4-1. Method

[1] Calu-3 cell line was inoculated in a 96-well plate and cultured overnight at 37°C.
[2] The Calu-3 cell line was cultured (pretreated) for 1 to 2 hours at 37°C in the presence of 11 present compounds (compound #1, compound #7, compound #9, compound #18, compound #19, compound #21, compound #22, compound #30, compound #13, compound #35 and compound #5) or molnupiravir at various concentrations (0.015242 µM, 0.045725 µM, 0.137174 µM, 0.411523 µM, 1.234568 µM, 3.703704 µM, 11.11111 µM, 33.33333 µM and 100 µM).
[3] Thereafter, the Calu-3 cell line was infected with SARS-CoV-2 (a prototype having D614G mutation) in the presence of the above 11 types of the present compounds or molnupiravir at MOI of 0.1 for 3 days.
[4] The infected Calu-3 cell line was fixed overnight in a solution containing 4% PFA.
[5] It was subjected to immunostaining using a monoclonal antibody against a SARS-CoV-2 N protein (bsm-41414M, manufactured by Bioss Antibodies) according to any conventional method to detect an infected cell.
[6] All cell nuclei were stained using Hoechst 33342 (manufactured by Thermo Fisher Scientific).
[7] A Cytation 5 cell imaging multimode plate reader (manufactured by BioTek) was used to acquire fluorescence images, and a CellProfiler image analysis software (manufactured by Broad Institute) was used to calculate the number of infected cells and total number of cells.
[8] GraphPad Prism (manufactured by GraphPad Software) was used to create graphs showing the relationships between the concentrations of the above 11 present compounds or molnupiravir, and the cell infection rate (%) and cell viability (%) (Figure 4-1 to Figure 4-3), and to calculate IC50 (Table 3).

### 4-2. Results

The results show that when the Calu-3 cell line was infected with SARS-CoV-2 (a prototype having D614G mutation) in the presence of the above 11 present compounds, the cell infection rate decreased in a concentration-dependent manner even if any of the present compounds was used, similarly to when molnupiravir was used (Figure 4-1 to Figure 4-3). The results of calculating IC50 confirmed that the above 11 present compounds exert the antiviral effect comparable to that of molnupiravir (Table 3). These results indicate that the present compounds other than compound #5 also have an antiviral action.

**[Table 3]**

| Test substance | IC50(µM) |
|---|---|
| Compound #1 | 21.4 |
| Compound #7 | 41.5 |
| Compound #9 | 8.0 |
| Compound #18 | 7.6 |
| Compound #19 | 4.1 |
| Compound #21 | 10.5 |
| Compound #22 | 2.0 |
| Compound #30 | 10.7 |
| Compound #13 | 1.1 |
| Compound #35 | 15.4 |
| Compound #5 | 2.6 |
| Molnupiravir | 2.9 |

### Example 5

### 5. Confirmation that present compound has an antiviral action against viruses other than coronavirus.

In order to confirm that the present compound has an antiviral action against viruses other than a coronavirus such as SARS-CoV-2, experiments were carried out according to the methods described under the following items "5-1. Method (type A influenza virus)" and "5-2. Method (Ebola virus)".

### 5-1. Method (type A influenza virus)

[1] Calu-3 cell line was inoculated in a 96-well plate and cultured overnight at 37°C.
[2] The Calu-3 cell line was cultured (pretreated) for 1 to 2 hours at 37°C in the presence of compound #5 or favipiravir (manufactured by FUJIFILM Corporation) at various concentrations (0.076 µM, 0.229 µM, 0.686 µM, 2.058 µM, 6.173 µM, 18.519 µM, 55.556 µM, 166.667 µM and 500 µM).
[3] Thereafter, the Calu-3 cell line was infected with type A influenza virus (H1N1 subtype) (A/Hokkaido/7/2016 MDCK(1)AH1pdm, distributed by the Hokkaido Institute of Public Health) in the presence of compound #5 or favipiravir at MOI of 0.1 for 2 days.
[4] The infected Calu-3 cell line was fixed overnight in a solution containing 10% formalin.
[5] It was subjected to immunostaining using a monoclonal antibody against NP protein (manufactured by Thermo Fisher Scientific) according to any conventional method to detect an infected cell.
[6] All cell nuclei were stained using Hoechst 33342 (manufactured by Thermo Fisher Scientific).
[7] A Cytation 5 cell imaging multimode plate reader (manufactured by BioTek) was used to acquire fluorescence images, and a CellProfiler image analysis software (manufactured by Broad Institute) was used to calculate the number of infected cells and total number of cells.
[8] GraphPad Prism (manufactured by GraphPad Software) was used to create graphs showing the relationships between the concentrations of compound #5 or favipiravir, and the cell infection rate (%) and cell viability (%) (Figure 5), and to calculate IC50 (Table 4).

### 5-2. Method (Ebola virus)

[1] A human liver-derived cell line (Huh7 cell line) expressing the NP/VP30/VP35/L proteins of Ebola virus (distributed by the Human Science Research Resources Bank) was inoculated in a 96-well plate and cultured overnight at 37°C.
[2] The Huh7 cell line was cultured (pretreated) for 1 to 2 hours at 37°C in the presence of compound #5 at various concentrations (1.95313 µM, 3.90625 µM, 7.8125 µM, 15.625 µM, 31.25 µM, 62.5 µM, 125 µM, 250 µM and 500 µM) or remdesivir (manufactured by Selleck Biotechnology) at various concentrations (0.00152 µM, 0.00457 µM, 0.01372 µM, 0.04115 µM, 0.12346 µM, 0.37037 µM, 1.11111 µM, 3.33333 µM and 10 µM).
[3] Thereafter, the Huh7 cell line was infected with Ebola virus-derived trVLP (produced using a plasmid distributed by the Friedrich-Loeffler Institute, Germany) in the presence of compound #5 or remdesivir for 2 days according to a conventional method.
[4] The infected Huh7 cell line was fixed overnight in a solution containing 10% formalin.
[5] All cell nuclei were stained using Hoechst 33342 (manufactured by Thermo Fisher Scientific).
[6] A Cytation 5 cell imaging multimode plate reader (manufactured by BioTek) was used to acquire fluorescence images, and a CellProfiler image analysis software (manufactured by Broad Institute) was used to calculate the number of infected cells and total number of cells.
[7] GraphPad Prism (manufactured by GraphPad Software) was used to create graphs showing the relationships between the concentrations of compound #5 or remdesivir, and the cell infection rate (%) and cell viability (%) (Figures 6), and to calculate IC50 (Table 4).

### 5-3. Results

The results show that both when the Calu-3 cell line was infected with type A influenza virus in the presence of compound #5 and when the Huh7 cell line was infected with type A influenza virus in the presence of compound #5, the cell infection rate decreased in a concentration-dependent manner with compound #5 (Figure 5 and Figure 6). The results of calculating IC50 confirmed that compound #5 exerted an antiviral effect, although at a higher concentration than the existing antiviral drug (favipiravir or remdesivir) (Table 4). These results indicate that the present compound has an antiviral action against a virus other than coronavirus (such as influenza virus or Ebola virus).

**[Table 4]**

| Virus | IC50(µM) | | |
|---|---|---|---|
| | Compound #5 | Favipiravir | Remdesivir |
| Type A influenza virus | 150 | 9.5 | - |
| Ebola virus | 202 | - | 0.1 |

### Industrial Applicability

The present invention is useful for the prevention or treatment of viral infections in the livestock industry and human medicine.

## Claims

1. An antiviral agent comprising one or more compounds selected from the group consisting of:
a compound of formula (I₀): 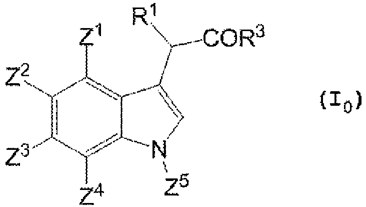
[wherein
R¹ represents
a hydrogen atom;
a benzoylmethyl group, the benzene ring of which is unsubstituted, or a benzoylmethyl group, the benzene ring of which is substituted with at least one group selected from the group consisting of an alkyl group having 1 to 7 carbon atoms, an alkoxy group having 1 to 7 carbon atoms, a fluorine atom and a chlorine atom;
a linear or branched alkyl group, having 4 to 6 carbon atoms, unsubstituted or substituted with a fluorine atom;
an alkyl group, having 1 to 6 carbon atoms, substituted with a group of formula (A): 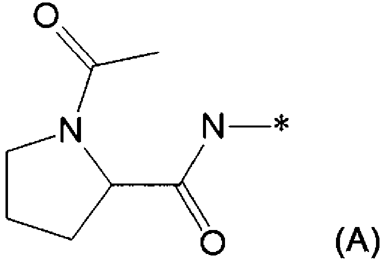
[wherein * indicates the bonding position] and optionally having an oxygen atom; or
a methyl group or an ethyl group, substituted with a phenyl group, a cyclopentyl group or a 1-acetyl-4-piperidinyl group, the phenyl group being optionally further substituted with one or more phenyl groups;
Z¹, Z², Z³ and Z⁴ are the same or different and each represent a hydrogen atom, a halogen atom, a C1-C6 alkyl group, a C2-C6 alkenyl group, a C2-C6 alkynyl group or a group of OR⁸ wherein R⁸ represents a C1-C7 alkyl group, a C2-C6 alkenyl group or a C2-C6 alkynyl group;
Z⁵ represents a hydrogen atom or a C1-C10 alkyl group; and
R³ represents any one group selected from the group consisting of OH, OR⁴, NHR⁴ and NR⁴R⁵, wherein R⁴ and R⁵ are the same or different and each represent a substituted or unsubstituted alkyl group having 1 to 4 carbon atoms];
a compound of formula (II): 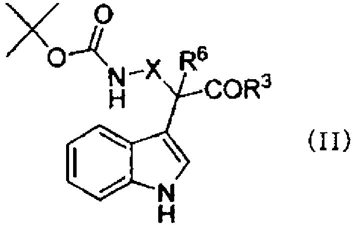
[wherein
R⁶ represents a hydrogen atom or a methyl group;
X represents an alkylene group, having 4 to 6 carbon atoms, optionally having an oxygen atom; and
R³ represents any one group selected from the group consisting of OH, OR⁴, NHR⁴ and NR⁴R⁵, wherein R⁴ and R⁵ are the same or different and each represent a substituted or unsubstituted alkyl group having 1 to 4 carbon atoms]; and
a compound of formula (III): 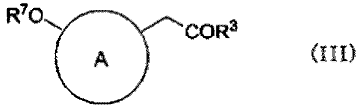
[wherein
A represents indole or naphthalene, and when A represents indole, it is substituted with a R³COCH₂ group and R⁷O at the 3-position and 5-position, respectively, and when A represents naphthalene, it is substituted with a R³COCH₂ group and R⁷O at the 1-position and 7-position, respectively;
R⁷ represents an alkyl group having 1 to 10 carbon atoms or a benzyl group, the benzene ring of which is optionally substituted with one or more of an alkyl group having 1 to 3 carbon atoms or an alkoxy group having 1 to 3 carbon atoms; and
R³ represents any one group selected from the group consisting of OH, OR⁴, NHR⁴ and NR⁴R⁵, wherein R⁴ and R⁵ are the same or different and each represent a substituted or unsubstituted alkyl group having 1 to 4 carbon atoms]; and pharmaceutically acceptable salts thereof.

2. The antiviral agent according to claim 1, wherein the compound is a compound of formula (I₀): 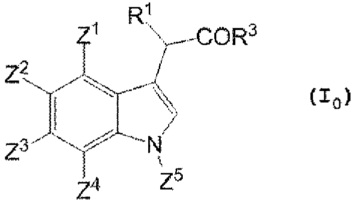 [wherein
R¹ represents
a hydrogen atom;
a benzoylmethyl group, the benzene ring of which is unsubstituted, or a benzoylmethyl group, the benzene ring of which is substituted with at least one group selected from the group consisting of an alkyl group having 1 to 7 carbon atoms, an alkoxy group having 1 to 7 carbon atoms, a fluorine atom and a chlorine atom;
a linear or branched alkyl group, having 4 to 6 carbon atoms, unsubstituted or substituted with a fluorine atom;
an alkyl group, having 1 to 6 carbon atoms, substituted with a group of formula (A): 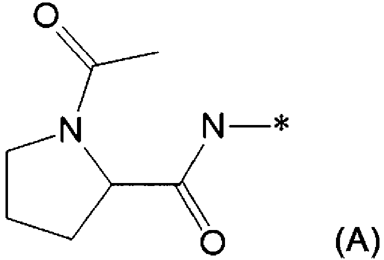
[wherein * indicates the bonding position] and optionally having an oxygen atom; or
a methyl group or an ethyl group, substituted with a phenyl group, a cyclopentyl group or a 1-acetyl-4-piperidinyl group, the phenyl group being optionally further substituted with one or more phenyl groups;
Z¹, Z², Z³ and Z⁴ are the same or different and each represent a hydrogen atom, a halogen atom, a C1-C6 alkyl group, a C2-C6 alkenyl group, a C2-C6 alkynyl group or a group of OR⁸ wherein R⁸ represents a C1-C7 alkyl group, a C2-C6 alkenyl group or a C2-C6 alkynyl group;
Z⁵ represents a hydrogen atom or a C1-C10 alkyl group; and
R³ represents any one group selected from the group consisting of OH, OR⁴, NHR⁴ and NR⁴R⁵, wherein R⁴ and R⁵ are the same or different and each represent a substituted or unsubstituted alkyl group having 1 to 4 carbon atoms]; or
a pharmaceutically acceptable salt thereof.

3. The antiviral agent according to claim 1, wherein the compound is a compound of formula (I-2), a compound of formula (I-3), a compound of formula (I-4), a compound of formula (I-5), a compound of formula (I-6), a compound of formula (I-7), a compound of formula (I-8), a compound of formula (I-9), a compound of formula (I-10), a compound of formula (II-2) or a compound of formula (III-2), or a pharmaceutically acceptable salt thereof.

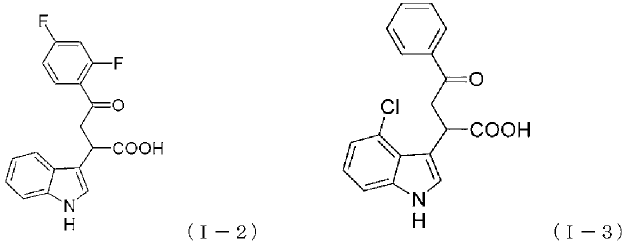

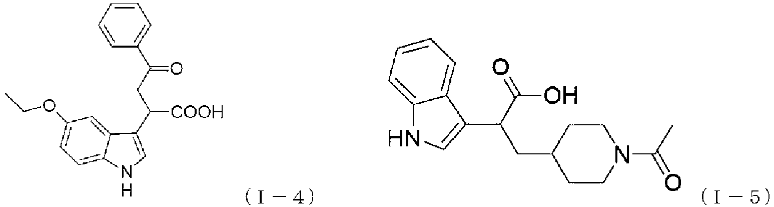

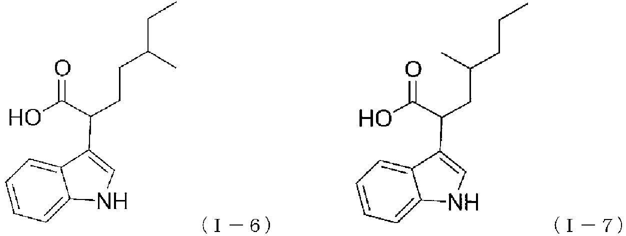

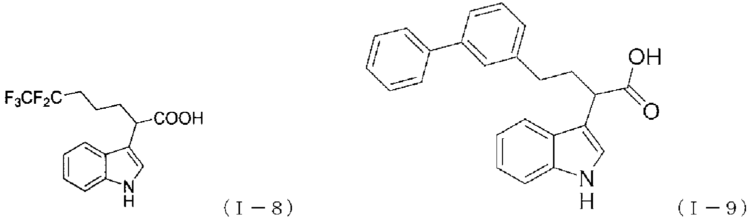

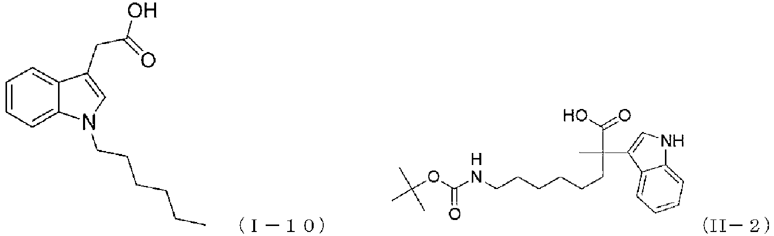

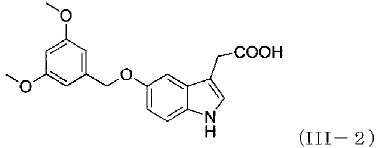

4. The antiviral agent according to any one of claims 1 to 3, wherein the virus is coronavirus, influenza virus or Ebola virus.
